# FASCICULE DE BREVET EUROPEEN

(11) **EP 1 072 236 B1**
(45) Date de publication et mention de la délivrance du brevet: **03.03.2004**
(21) Numéro de dépôt: 00420143.0
(22) Date de dépôt: 29.06.2000
(51) Int. Cl.: A61F 2/34

(54) **Cotyle pour prothèse de hanche**
Gelenkpfanne für Hüftprothese
Acetabular cup for hip prosthesis

(30) Priorité: 30.07.1999 FR 9910144
(43) Date de publication de la demande: 31.01.2001
(73) Titulaire: Biomet Merck France, 26000 Valence (FR); ORA, 69160 Tassin la Demi Lune (FR)
(72) Inventeur: Augoyard, Marc, 69160 Tassin La Demi Lune (FR); Bascoulergue, Gérard, Decédé (FR); Basso, Maurice, 83400 Hyeres (FR); Benedetto, Murielle, 26000 Valence (FR); Charret, Philippe, 69270 Fontaine sur Saone (FR); Courcelles, Philippe, Decédé (FR); Debiesse, Jean-Louis, 38200 Vienne (FR); Dupre Latour, Laurent, 42580 L'Etrat (FR); Eyraud, Guy, 38150 Ville Sous Anjou (FR); Fayard, Jean-Philippe, 42170 Saint Just Saint Rambert (FR); Hulin, Paul Henri, 89000 Auxerre (FR); Lecuire, François, 83400 Hyeres (FR); Melere, Gilles, 74000 Annecy (FR); Millon, Joseph, 73940 La Ravoire (FR); Passot, Jean-Paul, 42530 Saint Genest Lerpt (FR); Peyrot, Jacques, 69160 Tassin La Demi-Lune (FR); Relave, Marc, 42160 Andrezieux Boutheon (FR); De Witte, Gérard, 26300 Chateauneuf sur Isere (FR)
(74) Mandataire: Vuillermoz, Bruno

(56) Documents cités:
- EP-A- 0 118 194
- EP-A- 0 532 439
- EP-A- 0 743 050
- EP-A- 0 745 360
- EP-A- 0 900 552
- WO-A-97/42913
- DE-A- 19 731 442
- FR-A- 2 770 769
- US-A- 4 695 282

## Description

L'invention concerne un cotyle pour prothèse de hanche.

De manière connue, une prothèse de hanche comprend fondamentalement deux parties, respectivement un élément fémoral ou tige, destiné à être inséré dans le canal médullaire du fémur à prothéser, et portant à son extrémité libre une tête sphérique, et un cotyle, mis en place au niveau de la cavité cotyloïdienne de l'aile iliaque de l'articulation considérée, ce cotyle étant destiné à recevoir la tête sphérique dudit élément fémoral.

Plus précisément, ce cotyle est réalisé en deux parties distinctes, à savoir respectivement :
■ une cupule dite de « soutien », destinée à être insérée dans la cavité cotyloïdienne de l'aile iliaque ;
■ et un noyau ou insert, dit de frottement, destiné à s'insérer dans la cupule de soutien, et dont la face interne, de forme hémisphérique, est destinée à recevoir la tête sphérique de l'élément fémoral.

En pratique, la cupule de soutien est rigide et est réalisée le plus souvent en métal, tel que par exemple en un alliage de titane. En revanche, l'insert est le plus généralement réalisé en matière plastique, telle que notamment en polyéthylène haute densité, voire également en matière beaucoup plus dure, telle qu'en céramique, et notamment en alumine.

L'un des problèmes majeurs que l'on souhaite résoudre, dès lors que l'on met en oeuvre de tels cotyles, réside dans l'ancrage ferme et irréversible de la cupule de soutien au sein de la cavité cotyloïdienne de l'aile iliaque.

Comme par ailleurs, on souhaite s'affranchir de la mise en oeuvre de ciment afin d'éviter toute réaction de l'organisme vis à vis de ce dernier et par ailleurs, faciliter la mise en place desdites cupules, il est donc nécessaire de faire appel pour la mise en place dudit cotyle, d'une part à une fixation primaire, c'est à dire purement mécanique, et d'autre part, à une fixation secondaire, cette dernière étant de manière connue directement liée à la repousse osseuse.

Différentes solutions ont été proposées pour optimiser tant la fixation primaire que la fixation secondaire. On a par exemple proposé la mise en oeuvre d'une cupule, dont toute la base est munie d'excroissances, usinées dans la masse, propres à générer ainsi des saillies au niveau desquelles la repousse osseuse est optimisée, favorisant de la sorte l'ancrage (voir par exemple FR-A-2 770 769 et EP-A-0 743 050)

Le document DE-A-197 31 442 décrit un cotyle pour prothèse de hanche, dont la cupule de soutien présente une forme sphérique à triple rayons de courbure. Si certes, ces excroissances optimisent sans conteste possible une telle repousse osseuse, en revanche, elles génèrent un obstacle physique lors de l'insertion de la cupule par impactage au sein de la cavité cotyloïdienne, augmentant la difficulté pour le praticien lors de la pose d'une telle cupule.

L'objectif de l'invention est de s'affranchir de cet inconvénient.

Elle propose un cotyle du type en question, à la fois facile à réaliser et à mettre en place, et dont la structure favorise l'ancrage tant par fixation primaire que la repousse et partant, par fixation secondaire, augmentant ainsi de manière significative le maintien de la cupule dans ladite cavité cotyloïdienne.

Ce cotyle pour prothèse de hanche comprend :
■ une cupule de soutien destinée à être mise en place dans la cavité cotyloïdienne de l'aile iliaque de l'articulation à prothéser ;
■ un insert de frottement destiné :
   ◆ à être inséré dans la cupule de soutien ;
   ◆ et à recevoir la tête de l'élément fémoral de ladite prothèse.

Ce cotyle se caractérise :
■ en ce que la cupule de soutien présente un profil externe constitué d'une base conique se prolongeant par un secteur de calotte sphérique aplatie au niveau du pôle ;
■ et en ce que la base conique comporte des cannelures orientées selon des portions de méridien.

En d'autres termes, l'invention réside dans la combinaison d'un profil particulier associant une base en forme de tronc de cône avec une partie supérieure sphérique, avec la mise en oeuvre de cannelures, définissant un certain nombre de dents, orientées selon des méridiens, permettent tout à la fois de faciliter l'opération d'impactage de la cupule au sein de la cavité cotyloïdienne, et donc la fixation primaire, tout en optimisant la fixation secondaire de par la présence desdites cannelures.

Selon l'invention, la cupule présente sur sa surface externe une rainure s'étendant au sein des cannelures et perpendiculairement à la direction de celles-ci, et donc parallèlement à l'équateur de ladite cupule.

Selon une autre caractéristique de l'invention, la cupule présente une pluralité de gorges méridiennes, de faible profondeur, mais de largeur supérieure à la largeur des cannelures, lesdites gorges s'étendant du pôle et débouchant au niveau de la base de la cupule.

Selon une autre caractéristique de l'invention, la cupule présente en outre un orifice fileté, ménagé selon son axe de symétrie au niveau de l'extrémité supérieure de la calotte, donc au niveau du pôle, destiné à permettre le positionnement de l'ancillaire de pose.

Cet orifice fileté est susceptible d'être bouché par un bouchon vissé au niveau dudit filetage de telle sorte à aboutir à une continuité de la surface de la cupule, et en outre d'éviter le passage de débris, susceptibles d'être générés par le frottement entre la cupule et l'insert, et dont il a pu être montré qu'il est à l'origine d'ostéolyse, et partant de risque de descellement de la cupule.

Enfin, selon l'invention, la surface interne de la base de la cupule comporte des bossages destinés à coopérer avec un épaulement annulaire correspondant, ménagé au voisinage de la base du noyau et ce, afin de permettre le clipsage de celui-ci au sein de la cupule.

La manière dont l'invention peut être réalisée et les avantages qui en découlent ressortiront mieux de l'exemple de réalisation qui suit donné à titre indicatif et non limitatif à l'appui des figures annexées.

La figure 1 est une représentation schématique en perspective du cotyle conforme à l'invention.
La figure 2 est une vue latérale de la cupule conforme à l'invention, dont la figure 3 est une vue de détail de la base.
La figure 4 est une vue schématique du dessus de ladite cupule.
La figure 5 est une vue en section de la cupule.
La figure 6 est une vue en section transversale du noyau-insert de l'invention, dont la figure 7 une vue du dessous.
Les figures 8 et 9 sont des vues de détail de la coopération entre le noyau-insert et la cupule, respectivement avant et après encliquetage.

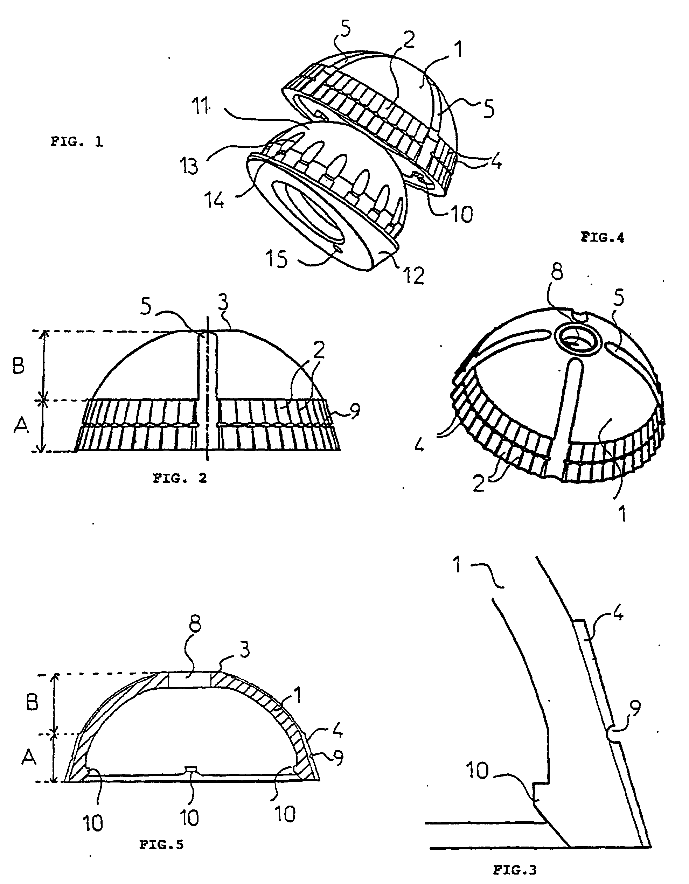

Le cotyle conforme à l'invention est constitué, ainsi que déjà précisé, d'une cupule de soutien désignée sous la référence générale (1), montrée en détail en relation avec les figures 2 à 5, destiné à recevoir un noyau-insert (11). Cette cupule est réalisée en métal, par exemple en acier inoxydable ou en titane.

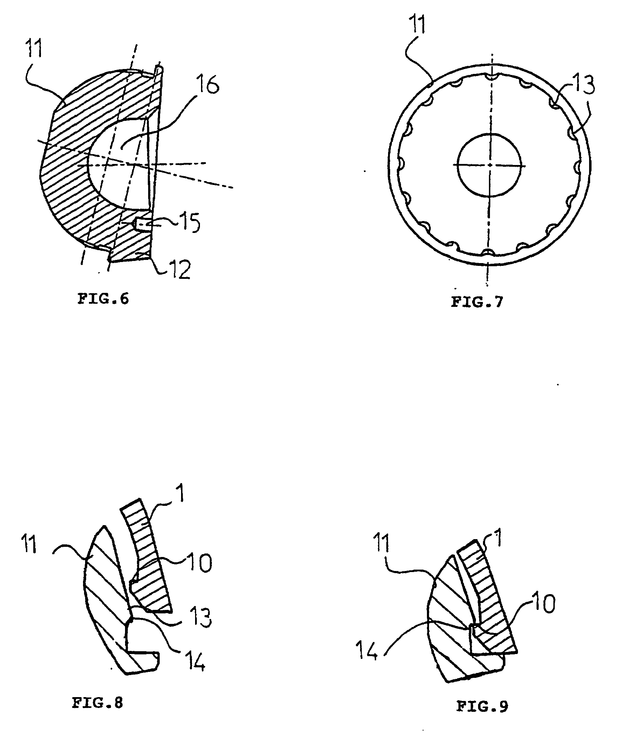

Elle présente une face externe convexe se composant d'une base conique s'étendant selon la hauteur A, se prolongeant par une calotte en forme de secteur sphérique s'étendant selon la hauteur B, dont l'extrémité supérieure est aplatie au niveau du pôle (3), ainsi qu'on peut bien l'observer sur les figures 2 et 5.

La forme particulière conique de la base A permet ainsi de favoriser l'impactage de la cupule au sein de la cavité cotyloïdienne et corollairement, d'optimiser la fixation primaire au niveau de celle-ci. En effet, la surépaisseur de la cupule à ce niveau inhérente à cette forme conique augmente sa rigidité à la base. Par ailleurs, compte tenu de cette conicité, il n'y a plus au niveau de la base de la cupule de convergence de ses extrémités l'une en direction de l'autre inhérente à la sphéricité des cupules traditionnelles, mais bien conservation de la divergence, optimisant de la sorte le caractère dénommé par l'homme du métier sous l'expression en langue anglaise « press-fit », se traduisant par une augmentation de la pression exercée par la base de la cupule au niveau de la cavité cotyloïdienne, et partant de la fixation primaire.

Ainsi qu'on peut l'observer sur les figures 2 et 3, la base conique de la cupule présente des cannelures (2) orientées selon des portions de méridien, chacune des cannelures (2) étant définie par deux dents en relief (4), s'étendant également selon des portions de méridien.

De la sorte, outre la réalisation d'aspérités inhérentes à la forme des cannelures, d'une profondeur typique de 0,6 mm, favorisant dès lors la repousse osseuse, donc la fixation secondaire, les dents (4) permettent également de s'opposer à la rotation de la cupule une fois mise en place au sein de la cavité cotyloïdienne.

Selon une caractéristique de l'invention, le fond de ces cannelures ne constitue pas de surépaisseur ou de saillie par rapport à l'enveloppe sphérique de base constituant la zone B. En d'autres termes, de par l'absence d'une telle saillie, on favorise l'introduction de ladite cupule au sein de la cavité cotyloïdienne lors de son impactage.

En outre, selon une autre caractéristique de l'invention, la pente du fond des cannelures n'est pas parallèle à la pente des dents (4) les définissant. En effet, et ainsi qu'on peut l'observer sur la figure 3, la pente des dents est supérieure à la pente du fond, de sorte que la hauteur des dents (4) au niveau de la jonction entre les zone A et B est supérieure à celle desdites dents au niveau de la base. Typiquement, cette hauteur varie de 0,6 mm à 0,4 mm.

Par ailleurs, les cannelures sont sensiblement divisées en deux par une rainure (9) parallèle à l'équateur, c'est à dire parallèle à la base de la cupule et donc, orientées perpendiculairement par rapport auxdites cannelures. Cette rainure a également pour objectif d'augmenter la surface développée de la cupule à ce niveau, et partant, d'optimiser la repousse osseuse une fois en place.

En outre et avantageusement, toute la base A de la cupule (1) est revêtue d'hydroxyapatite ou de phosphate de calcium, afin, et à titre surabondant, de favoriser l'ostéoconduction et donc d'optimiser la repousse osseuse.

Selon une autre caractéristique de l'invention, la cupule présente quatre gorges (5), s'étendant depuis le pôle (3) jusqu'à l'équateur à la base A de la cupule selon des méridiens, et débordant au niveau dudit équateur.

Ces gorges ont une profondeur voisine de 0,7 mm au niveau de la partie sphérique B et 1,2 mm au niveau de la partie conique A, et s'étendent sur une largeur supérieure à la largeur des cannelures (2) mentionnées précédemment. Elles sont destinées à évacuer les fluides graisseux, sanguins notamment provenant de l'os sous-chondrale après fraisage, lors de l'impactage de la cupule au niveau de la cavité cotyloïdienne. Ce faisant, on s'affranchit des emboles, notamment d'origine graisseuse, et des conséquences susceptibles d'en découler.

Ainsi que représenté sur la figure 4, ces gorges méridiennes (5) sont sensiblement réparties de manière équidistante, à 90 ° l'une de l'autre.

Selon une forme avantageuse de l'invention, le pôle (3) de la cupule est percé d'un orifice traversant (8), destiné à permettre la mise en oeuvre d'un ancillaire d'impactage. Cet orifice (8) est muni d'un filetage destiné à recevoir un bouchon vissable (non représenté), permettant ainsi de générer une continuité tant au niveau de la surface externe que de la surface interne de ladite cupule.

Ce faisant, cette continuité s'oppose à l'éventuel transfert de débris susceptible de survenir de par la coaptation du noyau - insert (11) avec la cupule (1) au niveau de la zone entre la cupule et l'os de la cavité au niveau de laquelle elle est implantée. On a en effet pu montrer que ces débris étaient susceptibles d'induire une ostéolyse locale et partant, le descellement de la dite cupule.

Ce bouchon ou pastille vissé est mis en place, bien entendu, après impaction de la cupule au sein de la cavité cotyloïdienne.

Ladite cupule présente au niveau de sa face interne des saillies ou ergots (10), dirigées radialement vers l'intérieur, et destinés à coopérer avec l'insert ou noyau de frottement (11) destiné à venir se clipser à l'intérieur de ladite cupule. Ces ergots ou saillies (10) sont par exemple au nombre de quatre, répartis de manière périodique tous les 90°.

Ce noyau - insert est décrit plus en détail en liaison avec les figures 1, 6 et 7. Il est typiquement réalisé en polyéthylène, ou en céramique, voire en selon la technique sandwich. Dans ce dernier cas, le noyau est constitué d'un insert en céramique, surmoulé par du polyéthylène. Ce surmoulage en polyéthylène permet d'obtenir un effet d'amortissement lorsque le cotyle est mis en place au sein de l'articulation. Après surmoulage, on procède à l'usinage du polyéthylène, afin de lui conférer sa configuration définitive. Afin en outre d'optimiser la cohésion entre la céramique et le polyéthylène, la surface externe de l'insert est pourvue d'aspérités.

Le noyau - insert (11) définit une cavité interne hémisphérique (16), destinée à recevoir la tête de l'élément fémorale d'une prothèse de hanche.

Ce noyau - insert est de facture classique et est pourvu d'un certain nombre d'encoches ou créneaux (13), régulièrement répartis sur tout le pourtour de la base du noyau, et s'étendant sensiblement selon des portions diamétrales, ces encoches étant limitées à la base inférieure de la partie hémisphérique dudit noyau. Ces encoches, par exemple au nombre de seize, sont destinées à coopérer avec les ergots (10) ménagés à l'intérieur de la cupule de soutien (1).

La base (12) du noyau peut être plate ou d'épaisseur variable. Dans cette hypothèse, elle tient compte bien entendu de son positionnement au sein de la cupule, fonction de l'orientation de l'articulation. De fait, cette épaisseur variable est destinée à augmenter le débattement de ladite articulation. De par le positionnement de la surépaisseur en position inférieure, on évite tout effet de came, qui, de manière connue, est susceptible de provoquer la luxation de l'articulation ainsi prothésée.

La base (12), lorsqu'elle est d'épaisseur variable comporte un orifice (15) borgne, dirigé sensiblement parallèlement à l'axe de révolution de l'insert, et destiné à permettre la mise en place de l'outil d'impactage lors de l'insertion du noyau au sein de la cupule.

Comme déjà indiqué, cet insert est destiné à être clipsé au sein de la cupule de soutien (1). Dans le cadre de la présente invention, ce clipsage intervient en l'absence de tout jonc métallique rapporté sur l'insert. Ce faisant, on s'affranchit d'une part des problèmes de clipsage, fréquemment rencontrés avec la mise en oeuvre d'un tel jonc, et en outre des phénomènes de bimétallisme susceptibles d'intervenir de par la mise en oeuvre de deux métaux différents, respectivement au niveau dudit jonc et de la cupule.

On a représenté sur les figures 8 et 9 le clipsage du noyau - insert (11) au sein de la cupule (1). Ainsi qu'on peut l'observer, les ergots (10) coopèrent avec l'épaulement ou le bourrelet (14) défini par la base (12) au niveau de chacune des encoches (13). On joue sur la capacité de déformation dudit bourrelet pour permettre le passage de l'ergot (10) pour assurer le clipsage du noyau à l'intérieur de ladite cupule.

Le cotyle conforme à l'invention s'avère simple à mettre en oeuvre et à réaliser. Par ailleurs, son ancrage est optimisé par le recours à une fixation primaire renforcée par le profil particulier de la cupule, et à une fixation secondaire optimisée de par la présence des cannelures.

## Revendications

1. Cotyle pour prothèse de hanche comprenant ;
■ une cupule de soutien (1) destinée à être mise en place dans la cavité cotyloïdienne de l'aile iliaque de l'articulation à prothéser ;
■ un insert ou noyau de frottement (11) destiné :
◆ à être inséré dans la cupule de soutien (1) ;
◆ et à recevoir la tête de l'élément fémoral de ladite prothèse ;
***caractérisé* :**
■ en ce que la cupule de soutien (1) présente un profil externe constitué d'une base conique A se prolongeant par un secteur B en forme de calotte sphérique aplatie au niveau du pôle (3) ;
■ et en ce que la base conique A comporte des cannelures (2) orientées selon des portions de méridien, lesdites cannelures étant définies par des dents (4), orientées également selon des portions de méridiens.

2. Cotyle pour prothèse de hanche selon la revendication 1, ***caractérisé* en ce que** la cupule de soutien (1) présente sur sa surface externe une rainure (9) s'étendant au sein des cannelures (2) et perpendiculairement à la direction de celles-ci, et donc parallèlement à l'équateur de ladite cupule.

3. Cotyle pour prothèse de hanche selon l'une des revendications 1 et 2, ***caractérisé* en ce que** la cupule de soutien (1) présente une pluralité de gorges méridiennes (5), de largeur supérieure à la largeur des cannelures (2), lesdites gorges s'étendant du pôle (3) jusqu'à la base de la cupule (1), et débouchant au niveau de l'équateur de ladite base.

4. Cotyle pour prothèse de hanche selon l'une des revendications 1 à 3, ***caractérisé* en ce que** le fond des cannelures (2) ne forme pas de surépaisseur ou de saillie par rapport à l'enveloppe sphérique de base constituant la zone B.

5. Cotyle pour prothèse de hanche selon l'une des revendications 1 à 4, ***caractérisé* en ce que** la pente du fond des cannelures (2) n'est pas parallèle à la pente des dents (4) les définissant, la pente desdites dents (4) étant supérieure à celle du fond des cannelures (2), de sorte que la hauteur des dents (4) au niveau de la jonction entre les zone A et B est supérieure à celle desdites dents au niveau de la base.

6. Cotyle pour prothèse de hanche selon l'une des revendications 1 à 5, ***caractérisé* en ce que** la cupule de soutien (1) présente en outre un orifice fileté (5), ménagé selon son axe de symétrie au niveau du pôle (3), et destiné à permettre le positionnement de l'ancillaire de pose.

7. Cotyle pour prothèse de hanche selon la revendication 6, ***caractérisé* en ce que** l'orifice fileté (5) est susceptible d'être bouché par un bouchon vissé au niveau dudit filetage de telle sorte à aboutir à une continuité de la surface de la cupule de soutien (1).

8. Cotyle pour prothèse de hanche selon l'une des revendications 1 à 7, ***caractérisé* en ce que** la surface interne de la base A de la cupule de soutien (1) comporte des bossages ou ergots (10) dirigés radialement, et destinés à coopérer avec un épaulement annulaire (14), ménagé au voisinage de la base (12) du noyau - insert (11), et plus spécifiquement au niveau d'encoches (13), orientées selon des portions de méridiens au niveau de la base dudit noyau - insert (11), et ce, afin de permettre le clipsage de celui-ci au sein de la cupule.

## Patentansprüche

1. Gelenkpfanne für eine Hüftprothese, umfassend:
• eine Stützschale (1), die dazu bestimmt ist, in der Gelenkhöhle des Hüftflügels des durch eine Prothese zu ersetzenden Gelenks angeordnet zu werden;
• einen Gleiteinsatz oder -kern (11), der dazu bestimmt ist:
- in die Stützschale (1) eingesetzt zu werden;
- und den Kopf des Oberschenkelelements der genannten Prothese aufzunehmen;
**dadurch gekennzeichnet:**
• **dass** die Stützschale (1) ein Außenprofil aufweist, das von einer konischen Basis A gebildet ist, das sich durch einen Sektor B in Form einer im Bereich des Pols (3) abgeflachten Kugelkappe verlängert;
• und **dass** die konische Basis A Rillen (2) aufweist, die entlang von Meridianabschnitten ausgerichtet sind, wobei die Rillen durch Zähne (4) definiert sind, die ebenfalls entlang der Meridianabschnitte ausgerichtet sind.

2. Gelenkpfanne für eine Hüftprothese nach Anspruch 1, **dadurch gekennzeichnet, dass** die Stützschale (1) auf ihrer Außenseite eine Nut (9) aufweist, die sich innerhalb der Rillen (2) und senkrecht zur Richtung derselben und somit parallel zum Teilungskreis der Schale erstreckt.

3. Gelenkpfanne für eine Hüftprothese nach einem der Ansprüche 1 und 2, **dadurch gekennzeichnet, dass** die Stützschale (1) eine Vielzahl von Meridiankehlen (5) mit einer größeren Breite als die Breite der Rillen (2) umfasst, wobei sich die genannten Kehlen vom Pol (3) bis zur Basis der Schale (1) erstrecken und auf Höhe des Teilungskreises der Basis münden.

4. Gelenkpfanne für eine Hüftprothese nach einem der Ansprüche 1 bis 3, **dadurch gekennzeichnet, dass** der Grund der Rillen (2) keine Überdicke oder Vorsprung in Bezug auf die Basiskugelhülle, die die Zone B darstellt, bildet.

5. Gelenkpfanne für eine Hüftprothese nach einem der Ansprüche 1 bis 4, **dadurch gekennzeichnet, dass** das Gefälle des Grundes der Rillen (2) nicht zum Gefälle der Zähne (4), die sie definieren, parallel ist, wobei das Gefälle der Zähne (4) größer als jenes des Grundes der Rillen (2) ist, so dass die Höhe der Zähne (4) im Bereich der Verbindung zwischen den Zonen A und B größer als jene der Zähne im Bereich der Basis ist.

6. Gelenkpfanne für eine Hüftprothese nach einem der Ansprüche 1 bis 5, **dadurch gekennzeichnet, dass** die Stützschale (1) ferner eine Gewindeöffnung (8) aufweist, die entlang ihrer Symmetrieachse im Bereich des Pols (3) vorgesehen und dazu bestimmt ist, die Positionierung des Hilfsgeräts zum Einsetzen zu ermöglichen.

7. Gelenkpfanne für eine Hüftprothese nach Anspruch 6, **dadurch gekennzeichnet, dass** die Gewindeöffnung (8) durch einen Stöpsel, der auf Höhe des Gewindes eingeschraubt ist, verschließbar ist, um eine Durchgängigkeit der Oberfläche der Stützschale (1) zu erzielen.

8. Gelenkpfanne für eine Hüftprothese nach einem der Ansprüche 1 bis 7, **dadurch gekennzeichnet, dass** die Innenfläche der Basis A der Stützschale (1) Buckel oder Vorsprünge (10) aufweist, die radial ausgerichtet und dazu bestimmt sind, mit einem ringförmigen Absatz (14), der in der Nähe der Basis (12) des Kerns - Einsatzes (11) und insbesondere im Bereich von Kerben (13), die entlang von Meridianabschnitten auf Höhe der Basis des Kerns - Einsatzes (11) ausgerichtet sind, zusammenzuwirken, um das Einschnappen desselben innerhalb der Schale zu ermöglichen.

## Claims

1. Acetabular cup for hip prosthesis, comprising:
- a support cup (1) intended to be placed in the acetabular cavity of the iliac crest of the joint to be fitted with the prosthesis;
- a friction insert or core (11) intended:
· to be inserted in the support cup (1);
· and to receive the head of the femoral element of said prosthesis;
**characterized in that**:
- the support cup (1) has an external profile formed by a conical base A which is continued by a sector B in the form of a spherical cap flattened at the area of the pole (3);
- and the conical base A has channels (2) oriented along meridian portions, said channel being defined by teeth (4) which are also oriented along meridian portions.

2. Acetabular cup for hip prosthesis according to Claim 1, **characterized in that** the support cup (1) has, on its outer surface, a groove (9) extending within the channels (2) and perpendicular to the direction of the latter, and therefore parallel to the equator of said cup.

3. Acetabular cup for hip prosthesis according to either of Claims 1 and 2, **characterized in that** the support cup (1) has a plurality of meridional recesses (5) with a width greater than the width of the channels (2), said recesses extending from the pole (3) to the base of the cup (1), and opening out in the area of the equator of said base.

4. Acetabular cup for hip prosthesis according to one of Claims 1 to 3, **characterized in that** the bottom of the channels (2) does not form an excess thickness or protrusion relative to the spherical base envelope constituting zone B.

5. Acetabular cup for hip prosthesis according to one of Claims 1 to 4, **characterized in that** the slope of the bottom of the channels (2) is not parallel to the slope of the teeth (4) defining them, the slope of said teeth (4) being greater than that of the bottom of the channels (2), so that the height of the teeth (4) at the area of the junction between zones A and B is greater than that of said teeth at the area of the base.

6. Acetabular cup for hip prosthesis according to one of Claims 1 to 5, **characterized in that** the support cup (1) additionally has a threaded orifice (8) formed on its axis of symmetry at the area of the pole (3) and intended to permit positioning of the implantation ancillary.

7. Acetabular cup for hip prosthesis according to Claim 6, **characterized in that** the threaded orifice (8) is able to be plugged with a stopper screwed into said thread so as to obtain a continuity with the surface of the support cup (1).

8. Acetabular cup for a hip prosthesis according to one of Claims 1 to 7, **characterized in that** the inner surface of the base A of the support cup (1) comprises bosses or studs (10) directed radially and intended to cooperate with an annular shoulder (14) formed in proximity to the base (12) of the insert core (11), and more specifically in the area of notches (13) which are oriented along meridian portions at the area of the base of said insert core (11), in order to allow the latter to be clipped inside the cup.
